# EUROPÄISCHE PATENTANMELDUNG

(11) **EP 3 184 645 A1**
(43) Veröffentlichungstag der Anmeldung: **28.06.2017**
(21) Anmeldenummer: 15201813.1
(22) Anmeldetag: 22.12.2015
(51) Int. Cl.: C12Q 1/68

(54) **DIAGNOSTISCHE MIRNA SIGNATUREN IN MS UND CIS PATIENTEN**

(71) Anmelder: Siemens Healthcare GmbH, 91052 Erlangen (DE)
(72) Erfinder: Keller, Andreas, 66346 Püttlingen (DE); Stähler, Cord Friedrich, 69493 Hirschberg an der Bergstraße (DE); Geisen, Stefanie, 66125 Saarbrücken (DE); Sickert, Daniel, 90403 Nürnberg (DE)

(57) **Zusammenfassung**

Die Erfindung betrifft ein Verfahren zur Diagnose von multipler Sklerose in einem Patienten, bei dem das Expressionsprofils von miRNAs in einer Blutprobe bestimmt wird und ein Vergleich der Expressionsniveaus definierter miRNAs zu einer Referenzprobe durchgeführt wird, sowie die Verwendung dieser definierten miRNAs als diagnostische Marker für multiple Sklerose, und ein Kit zur Diagnose von multipler Sklerose.

## Beschreibung

Die Erfindung betrifft ein Verfahren zur Diagnose von multipler Sklerose (MS) in einem Patienten, bei dem das Expressionsprofils von miRNAs in einer Blutprobe bestimmt wird und ein Vergleich der Expressionsniveaus definierter miRNAs zu einer Referenzprobe durchgeführt wird, sowie die Verwendung dieser definierten miRNAs als diagnostische Marker für multiple Sklerose, und ein Kit zur Diagnose von multipler Sklerose.

Eine gesicherte Diagnose der Multiplen Sklerose kann - besonders im Frühstadium der Erkrankung - mit hohem Aufwand verbunden sein: Sind Symptome und klinischer Befund nicht eindeutig, werden neben der neurologischen Untersuchung in der Regel MRI, Lumbalpunktion und Messung der Nervenreaktionszeit mit visuell evozierten Potenzialen (VEP), akustisch evozierten Potenzialen (AEP) und somatosensibel evozierten Potenzialen (SEP) notwendig (Compston, A. & Coles, A. (2008). Multiple sclerosis. The Lancet, 372(9648), 1502-1517. doi:10.1016/S0140-6736(08)61620-7).

Patienten, die eine CIS (clinically isolated syndrome, klinisch isoliertes Syndrom) Diagnose erhalten, werden in vielen Fällen (nach einem weiteren Schub / einer neuen Läsionen, entsprechend den McDonald Kriterien (McDonald, W.I., Compston, A., Edan, G., Goodkin, D., Härtung, H.P., Lublin, F.D., Wolinsky, J.S. (2001). Recommended diagnostic criteria for multiple sclerosis: Guidelines from the International Panel on the Diagnosis of Multiple Sclerosis. Annals of Neurology, 50(1), 121-127. doi:10.1002/ana.1032)) im weiteren Verlauf des Lebens als RRMS (relapsing-remitting multiple sclerosis, schubförmig remittierende multiple Sklerose) Patienten eingestuft.

Für eine schnelle und effektive Behandlung von multipler Sklerose ist es vorteilhaft, wenn eine schnelle Diagnosemethode, insbesondere für CIS-Patienten, vorhanden ist. Es ist insbesondere vorteilhaft, wenn differentialdiagnostisch zwischen dem CIS-Stadium und einer bereits eingetretenen RRMS-Erkrankung unterschieden werden kann, bevor sämtliche bisher bekannten Kriterien einer RRMS erfüllt sind.

In letzter Zeit wurden microRNAs (nachfolgend auch miRNAs) als Marker für die Diagnose von verschiedenen Erkrankungen wie Lungenkrebs, etwa in der EP 2 438 190 B1, oder für das akute Koronarsyndrom, etwa in der EP 2 561 091, diskutiert. MicroRNAs sind hierbei eine Klasse von kurzen, nichtkodierenden RNAs, die beispielsweise eine Rolle bei der Genexpression spielen.

Es ist eine Aufgabe der Erfindung, ein miRNA Profil aus dem Blut von Patienten herauszuarbeiten, welches eine Diagnose (CIS vs. RRMS) vereinfachen kann.

Die Erfinder haben herausgefunden, dass mithilfe einer Änderung im Expressionsprofil von bestimmten miRNAs im Vergleich zu CIS Patienten, also solchen, bei denen kein Übergang zu multipler Sklerose, insbesondere RRMS, stattgefunden hat, eine Diagnose einer Erkrankung mit multipler Sklerose möglich ist, insbesondere bei CIS-Patienten.

Gemäß einem ersten Aspekt betrifft die Erfindung ein Verfahren zur Diagnose von multipler Sklerose in einem Patienten, umfassend:
Bereitstellen einer Blutprobe eines Patienten,
Erstellen eines Expressionsprofils von miRNAs aus der Blutprobe, und
Vergleich des Expressionsniveaus mindestens einer miRNA, die eine Sequenz umfasst, die ausgewählt ist aus der Gruppe, bestehend aus hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5p, bevorzugt hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-5011-3p, hsa-miR-6778-3p, und hsa-miR-6873-5p, weiter bevorzugt hsa-miR-181d-5p, hsa-miR-194-5p, hsa-miR-627-5p, hsa-miR-148a-5p, hsa-miR-4465, hsa-miR-4699-3p, hsa-miR-5011-3p, und hsa-miR-6873-5p, und/oder die eine Sequenz umfasst, die von diesen Sequenzen um jeweils bis zu 3 Basen, bevorzugt bis zu 2 Basen, weiter bevorzugt 1 Base abweicht, mit einem Referenzniveau, wobei der Vergleich die Diagnose einer Erkrankung mit multipler Sklerose ermöglicht.

Gemäß einem weiteren Aspekt betrifft die Erfindung eine Verwendung einer miRNA, die eine Sequenz umfasst, die ausgewählt ist aus der Gruppe, bestehend aus hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5p, bevorzugt hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-5011-3p, hsa-miR-6778-3p, und hsa-miR-6873-5p, weiter bevorzugt hsa-miR-181d-5p, hsa-miR-194-5p, hsa-miR-627-5p, hsa-miR-148a-5p, hsa-miR-4465, hsa-miR-4699-3p, hsa-miR-5011-3p, und hsa-miR-6873-5p, und/oder die eine Sequenz umfasst, die von diesen Sequenzen um jeweils bis zu 3 Basen, bevorzugt bis zu 2 Basen, weiter bevorzugt 1 Base abweicht, als diagnostischer Marker für die Diagnose von multipler Sklerose, insbesondere RRMS.

Zudem betrifft die Erfindung ein Kit zur Diagnose von multipler Sklerose in einer Blutprobe eines Patienten, umfassend: Sonden und/oder Primer zur Detektion mindestens einer miRNA-Sequenz, die eine Sequenz umfasst, die ausgewählt ist aus der Gruppe, bestehend aus hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5p, bevorzugt hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-5011-3p, hsa-miR-6778-3p, und hsa-miR-6873-5p, weiter bevorzugt hsa-miR-181d-5p, hsa-miR-194-5p, hsa-miR-627-5p, hsa-miR-148a-5p, hsa-miR-4465, hsa-miR-4699-3p, hsa-miR-5011-3p, und hsa-miR-6873-5p, und/oder die eine Sequenz umfasst, die von diesen Sequenzen um jeweils bis zu 3 Basen, bevorzugt bis zu 2 Basen, weiter bevorzugt 1 Base abweicht.

Weitere Aspekte der vorliegenden Erfindung sind den abhängigen Ansprüchen und der detaillierten Beschreibung zu entnehmen.

Sofern nicht anderweitig definiert haben hierin verwendete technische und wissenschaftliche Begriffe dieselbe Bedeutung, wie sie im Allgemeinen von einem Fachmann im Gebiet der Erfindung verstanden wird.

MicroRNAs bzw. miRNAS bzw. micro-Ribonukleinsäuren sind eine Klasse von kurzen, nichtkodierenden RNAs, die beispielsweise eine Rolle bei der Genexpression spielen. Eine miRNA ist hierbei ein Polynucleotid mit einer festen Anzahl von Basen, beispielsweise von weniger als 500, weniger als 200, weniger als 100, weniger als 50 oder weniger als 30, aber beispielsweise mehr als 5, 10 oder 14 Basen, und einer definierten Sequenz. Aus dieser kann beispielsweise auch cDNA gewonnen werden, so dass aus den miRNAs im erfindungsgemäßen Verfahren auch cDNAs hergestellt werden können, welche dann ebenfalls mit cDNA-Niveaus verglichen werden können, die aus miRNAs von Patienten gewonnen werden, welche keine MS, insbesondere keine RRMS, aufweisen.

Im Folgenden wie auch zuvor beziehen sich die Verweise auf bestimmte miRNAs auf die Sequenzen, wie sie der Datenbank Mirbase (http://mirbase.org/), insbesondere Mirbase20 - also der Version 20 von Mirbase, entnommen werden können.

Des Weiteren sind die Sequenzen, die im erfindungsgemäßen Verfahren sowie der erfindungsgemäßen Verwendung Anwendung finden, auch der folgenden Tabelle 1 zu entnehmen. In Tabelle 1 sind hierbei die miRNAs in der reifen Form (mature Form) angegeben, welche von der Haarnadelform (stem loop form) abweichen kann.

**Tabelle 1: Sequenzen der miRNAs, die im erfindungsgemäßen Verfahren sowie der erfindungsgemäßen Verwendung Anwendung finden**

| **Reife Form** | **SEQ ID NO:** | **Sequenz** |
|---|---|---|
| | | |
| hsa-miR-194-5p | 1 | uguaacagcaacuccaugugga |
| hsa-miR-627-5p | 2 | gugagucucuaagaaaagagga |
| hsa-miR-148a-5p | 3 | aaaguucugagacacuccgacu |
| hsa-miR-1181 | 4 | ccgucgccgccacccgagccg |
| hsa-miR-1273g-3p | 5 | accacugcacuccagccugag |
| hsa-miR-181d-5p | 6 | aacauucauuguugucggugggu |
| hsa-miR-4465 | 7 | cucaaguagucugaccagggga |
| hsa-miR-4699-3p | 8 | aauuuacucugcaaucuucucc |
| hsa-miR-4715-3p | 9 | gugccaccuuaacugcagccaau |
| hsa-miR-5011-3p | 10 | gugcauggcuguauauauaaca |
| hsa-miR-6778-3p | 11 | ugccucccugacauuccacag |
| hsa-miR-6833-5p | 12 | guguggaagaugggaggagaaa |
| hsa-miR-6873-5p | 13 | cagagggaauacagagggcaau |

Die vorliegende Erfindung betrifft in einem ersten Aspekt ein Verfahren zur Diagnose von multipler Sklerose in einem Patienten, umfassend:
Bereitstellen einer Blutprobe eines Patienten,
Erstellen eines Expressionsprofils von miRNAs aus der Blutprobe, und
Vergleich des Expressionsniveaus mindestens einer miRNA, die eine Sequenz umfasst, die ausgewählt ist aus der Gruppe, bestehend aus hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5p, bevorzugt hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-5011-3p, hsa-miR-6778-3p, und hsa-miR-6873-5p, weiter bevorzugt hsa-miR-181d-5p, hsa-miR-194-5p, hsa-miR-627-5p, hsa-miR-148a-5p, hsa-miR-4465, hsa-miR-4699-3p, hsa-miR-5011-3p, und hsa-miR-6873-5p, und/oder die eine Sequenz umfasst, die von diesen Sequenzen um jeweils bis zu 3 Basen, bevorzugt bis zu 2 Basen, weiter bevorzugt 1 Base abweicht, mit einem Referenzniveau, wobei der Vergleich die Diagnose einer Erkrankung mit multipler Sklerose ermöglicht.

Das Expressionsprofil ist hierbei ein Profil der Expression von miRNAs, beispielsweise 1, 2, 3, 4, 5, 6, 7, 8, 9, 10, 11, 12, 13, 14, 15, 16, 17, 18, 19, 20, 30, 40, 50, 60, 70, 80, 90, 100 oder mehr miRNAs, in der Probe, gemäß bestimmten Ausführungsformen in quantitativer Form, während das Expressionsniveau die jeweilige Menge einer exprimierten miRNA angibt.

Das Bereitstellen der Blutprobe ist hierbei nicht besonders beschränkt, ist insbesondere jedoch nichtinvasiv, sondern es wird eine bereits genommene Blutprobe verwendet. Gemäß bestimmten Ausführungsformen kann auch die Entnahme und/oder Aufbereitung einer Blutprobe umfasst sein. Eine Blutprobe umfasst hierbei eine Vollblutprobe wie auch Fraktionen von Blutproben wie etwa das Plasma, Serum, etc., oder auch Zellen in der Blutprobe wie mononukleäre Zellen des peripheren Blutes.

Der Patient, von dem die Blutprobe bereitgestellt wird, ist ebenfalls nicht besonders beschränkt, und kann beispielsweise Wirbeltiere, insbesondere Säugetiere umfassen, ist jedoch gemäß bestimmten Ausführungsformen ein Mensch. Insbesondere wird das vorliegende Verfahren gemäß bestimmten Ausführungsformen bei Patienten angewendet, z.B. Menschen, bei denen ein klinisch isoliertes Syndrom bzw. CIS diagnostiziert wurde. Die Sequenzen, die im erfindungsgemäßen Verfahren zur Anwendung kommen, können hierbei insbesondere bei einer Veränderung des Expressionsniveaus einer oder mehrerer der miRNAs, die eine entsprechende Sequenz umfassen, zur Anwendung kommen.

Auch ist das Verfahren zum Erstellen eines Expressionsprofils von miRNAs nicht besonders beschränkt und kann hierbei beispielsweise gängige mikrobiologische Verfahren zur Bestimmung von Nukleinsäuren umfassen, wobei hier zum Vergleich mit dem Referenzniveau gemäß bestimmten Ausführungsformen ein semiquantitatives oder quantitatives, insbesondere quantitatives Verfahren zur Anwendung kommt. Gemäß bestimmten Ausführungsformen umfasst das Erstellen des Expressionsprofils von miRNAs mindestens ein Verfahren, dass ausgewählt ist aus der Gruppe, umfassend eine Nukleinsäurehybridisierung, eine Nukleinsäureamplifikation, eine Polymeraseextension, eine Sequenzierung, eine Massenspektrometrie, und jegliche Kombination davon.

Eine Nukleinsäurehybridisierung kann hierbei die Verwendung von Arrays, z.B. Mikroarrays, und/oder eine *in situ* Hybridisierung umfassen. Ein Array kann dazu beispielsweise die Verwendung komplementärer Sequenzen zu den bestimmten Sequenzen, also miRNAs, die eine Sequenz umfassen, die ausgewählt ist aus der Gruppe, bestehend aus hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5p, bevorzugt hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-5011-3p, hsa-miR-6778-3p, und hsa-miR-6873-5p, weiter bevorzugt hsa-miR-181d-5p, hsa-miR-194-5p, hsa-miR-627-5p, hsa-miR-148a-5p, hsa-miR-4465, hsa-miR-4699-3p, hsa-miR-5011-3p, und hsa-miR-6873-5p, und/oder die eine Sequenz umfassen, die von diesen Sequenzen um jeweils bis zu 3 Basen, bevorzugt bis zu 2 Basen, weiter bevorzugt 1 Base abweicht, umfassen, welche beispielsweise geeignet auf einen Träger angebracht werden können. Für eine Quantifizierung kann dann beispielsweise eine Markierung der miRNAs der Probe mit Markern wie Fluoreszenzmarkern oder Radionukleotidmarkern erfolgen.

Alternativ oder zusätzlich kann auch eine Polymerase-Kettenreaktion (PCR, polymerase chain reaction), bevorzugt eine quantitative PCR (qPCR), insbesondere eine quantitative Echtzeit-PCR (qRT-PCR, quantitative real-time PCR) auf geeignete Weise durchgeführt werden.

Auch sind Sequenzierungsmethoden wie Next Generation Sequenzing und/oder Massenspektrometrie, insbesondere quantitative Massenspektrometrie, denkbar.

Der Vergleich mit einem Referenzniveau im erfindungsgemäßen Verfahren ist nicht besonders beschränkt und kann hierbei insbesondere einen Vergleich mit der Probe eines gesunden Individuums und/oder eines Individuums mit einer CIS-Diagnose, bei dem im Anschluss jedoch keine MS mehr auftritt und/oder eines Individuums, bei dem sich aus einer CIS-Diagnose eine MS, insbesondere RRMS entwickelt, umfassen. Durch solch einen Vergleich kann beispielsweise die Diagnose einer Erkrankung mit multipler Sklerose ermöglicht werden. Ein Vergleich kann hierbei beispielsweise anhand mathematischer Ansätze wie Korrelationen, anhand statistischer Verfahren, anhand Wahrscheinlichkeitstheorien, anhand informationstheoretischer Ansätze oder Kombinationen davon stattfinden.

Für eine verbesserte Aussage ist hierbei ein statistischer Vergleich mit einer Vielzahl von Proben von gesunden und an MS, insbesondere RRMS, erkrankten Patienten vorteilhaft, wobei solche Daten auch beispielsweise einer geeigneten Datenbank entnommen werden können und/oder anhand einer daraus entwickelten mathematischen Funktion bzw. eines Algorithmus bestimmt werden können. Das statistische Verfahren ist hierbei nicht besonders beschränkt und kann beispielsweise auch computergestützt durchgeführt werden. Beispielsweise können hierbei ein t-Test, ein Wilcoxon-Mann-Whitney-Test, die Bestimmung der Fläche unter der Kurve (AUC, area under curve), etc. zur Anwendung kommen.

Gemäß bestimmten Ausführungsformen wird das Referenzniveau aus einer Vielzahl von Proben von Patienten erhalten, von denen eine erste Gruppe in einer ersten und zweiten Diagnose mit CIS diagnostiziert wurde, eine zweite Gruppe nach einer ersten CIS-Diagnose in einer zweiten Diagnose mit RRMS diagnostiziert wurde, und eine dritte Gruppe in einer ersten und zweiten Diagnose mit RRMS diagnostiziert wurde. Gemäß bestimmten Ausführungsformen wird hierbei ein Referenzniveau aus derselben Art von Probe bestimmt wie die zu bestimmende Probe.

Als Abweichung von der Sequenz ist hierbei eine Variation in der Sequenz, z.B. eine Addition,, eine Insertion, eine Deletion, oder ein Austausch einer Base in der Sequenz im Vergleich zur ursprünglichen Sequenz, welche beispielsweise in Tabelle 1 angegeben ist, zu verstehen, insbesondere eine Deletion und/oder ein Einzelbasenaustausch. So kann beispielsweise eine Deletion von 1 oder 2 Basen an einem und/oder beiden Enden der Sequenz stattfinden, wobei maximal 3 Basen, bevorzugt maximal 2 Basen, weiter bevorzugt 1 Base deletiert werden und insbesondere keine Base deletiert wird. Auch ist zusätzlich dazu oder alternativ eine Änderung von 1 oder mehr Basen innerhalb der Sequenz möglich, wobei wiederum maximal eine Änderung um 3 Basen, bevorzugt 2 Basen, weiter bevorzugt 1 Base und insbesondere bevorzugt keiner Base im Vergleich zu den Sequenzen von hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5p, bevorzugt hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-5011-3p, hsa-miR-6778-3p, und hsa-miR-6873-5p, weiter bevorzugt hsa-miR-181d-5p, hsa-miR-194-5p, hsa-miR-627-5p, hsa-miR-148a-5p, hsa-miR-4465, hsa-miR-4699-3p, hsa-miR-5011-3p, und hsa-miR-6873-5p stattfindet.

Gemäß bestimmten Ausführungsformen findet also ein Vergleich des Expressionsniveaus mindestens einer miRNA, die eine Sequenz umfasst, die ausgewählt ist aus der Gruppe, bestehend aus hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5p, bevorzugt hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-5011-3p, hsa-miR-6778-3p, und hsa-miR-6873-5p, weiter bevorzugt hsa-miR-181d-5p, hsa-miR-194-5p, hsa-miR-627-5p, hsa-miR-148a-5p, hsa-miR-4465, hsa-miR-4699-3p, hsa-miR-5011-3p, und hsa-miR-6873-5p, mit einem Referenzniveau statt.

Gemäß bestimmten Ausführungsformen findet mindestens ein Vergleich mit einer Sequenz umfassend die miRNA hsa-miR-181d-5p und/oder eine Sequenz, die von dieser Sequenz, also der Sequenz der miRNA, um bis zu 3 Basen, bevorzugt bis zu 2 Basen, weiter bevorzugt 1 Base abweicht, statt. Gemäß bestimmten Ausführungsformen findet mindestens ein Vergleich mit einer Sequenz umfassend die miRNA hsa-miR-194-5p und/oder eine Sequenz, die von dieser Sequenz, um bis zu 3 Basen, bevorzugt bis zu 2 Basen, weiter bevorzugt 1 Base abweicht, statt. Gemäß bestimmten Ausführungsformen findet mindestens ein Vergleich mit einer Sequenz umfassend die miRNA hsa-miR-627-5p und/oder eine Sequenz, die von dieser Sequenz, um bis zu 3 Basen, bevorzugt bis zu 2 Basen, weiter bevorzugt 1 Base abweicht, statt. Gemäß bestimmten Ausführungsformen findet mindestens ein Vergleich mit einer Sequenz umfassend die miRNA hsa-miR-148a-5p und/oder eine Sequenz, die von dieser Sequenz, um bis zu 3 Basen, bevorzugt bis zu 2 Basen, weiter bevorzugt 1 Base abweicht, statt. Gemäß bestimmten Ausführungsformen findet mindestens ein Vergleich mit einer Sequenz umfassend die miRNA hsa-miR-1181 und/oder eine Sequenz, die von dieser Sequenz, um bis zu 3 Basen, bevorzugt bis zu 2 Basen, weiter bevorzugt 1 Base abweicht, statt. Gemäß bestimmten Ausführungsformen findet mindestens ein Vergleich mit einer Sequenz umfassend die miRNA hsa-miR-1273g-3p und/oder eine Sequenz, die von dieser Sequenz um bis zu 3 Basen, bevorzugt bis zu 2 Basen, weiter bevorzugt 1 Base abweicht, statt. Gemäß bestimmten Ausführungsformen findet mindestens ein Vergleich mit einer Sequenz umfassend die miRNA hsa-miR-4465 und/oder eine Sequenz, die von dieser Sequenz um bis zu 3 Basen, bevorzugt bis zu 2 Basen, weiter bevorzugt 1 Base abweicht, statt. Gemäß bestimmten Ausführungsformen findet mindestens ein Vergleich mit einer Sequenz umfassend die miRNA hsa-miR-4699-3p und/oder eine Sequenz, die von dieser Sequenz um bis zu 3 Basen, bevorzugt bis zu 2 Basen, weiter bevorzugt 1 Base abweicht, statt. Gemäß bestimmten Ausführungsformen findet mindestens ein Vergleich mit einer Sequenz umfassend die miRNA hsa-miR-4715-3p und/oder eine Sequenz, die von dieser Sequenz um bis zu 3 Basen, bevorzugt bis zu 2 Basen, weiter bevorzugt 1 Base abweicht, statt. Gemäß bestimmten Ausführungsformen findet mindestens ein Vergleich mit einer Sequenz umfassend die miRNA hsa-miR-5011-3p und/oder eine Sequenz, die von dieser Sequenz um bis zu 3 Basen, bevorzugt bis zu 2 Basen, weiter bevorzugt 1 Base abweicht, statt. Gemäß bestimmten Ausführungsformen findet mindestens ein Vergleich mit einer Sequenz umfassend die miRNA hsa-miR-6778-3p und/oder eine Sequenz, die von dieser Sequenz um bis zu 3 Basen, bevorzugt bis zu 2 Basen, weiter bevorzugt 1 Base abweicht, statt. Gemäß bestimmten Ausführungsformen findet mindestens ein Vergleich mit einer Sequenz umfassend die miRNA hsa-miR-6833-5p und/oder eine Sequenz, die von dieser Sequenz um bis zu 3 Basen, bevorzugt bis zu 2 Basen, weiter bevorzugt 1 Base abweicht, statt. Gemäß bestimmten Ausführungsformen findet mindestens ein Vergleich mit einer Sequenz umfassend die miRNA hsa-miR-6873-5p und/oder eine Sequenz, die von dieser Sequenz um bis zu 3 Basen, bevorzugt bis zu 2 Basen, weiter bevorzugt 1 Base abweicht, statt.

Gemäß bestimmten Ausführungsformen findet mindestens ein Vergleich mit einer Sequenz umfassend die miRNA hsa-miR-181d-5p statt. Gemäß bestimmten Ausführungsformen findet mindestens ein Vergleich mit einer Sequenz umfassend die miRNA hsa-miR-194-5p statt. Gemäß bestimmten Ausführungsformen findet mindestens ein Vergleich mit einer Sequenz umfassend die miRNA hsa-miR-627-5p statt. Gemäß bestimmten Ausführungsformen findet mindestens ein Vergleich mit einer Sequenz umfassend die miRNA hsa-miR-148a-5p statt. Gemäß bestimmten Ausführungsformen findet mindestens ein Vergleich mit einer Sequenz umfassend die miRNA hsa-miR-1181 statt. Gemäß bestimmten Ausführungsformen findet mindestens ein Vergleich mit einer Sequenz umfassend die miRNA hsa-miR-1273g-3p statt. Gemäß bestimmten Ausführungsformen findet mindestens ein Vergleich mit einer Sequenz umfassend die miRNA hsa-miR-4465 statt. Gemäß bestimmten Ausführungsformen findet mindestens ein Vergleich mit einer Sequenz umfassend die miRNA hsa-miR-4699-3p statt. Gemäß bestimmten Ausführungsformen findet mindestens ein Vergleich mit einer Sequenz umfassend die miRNA hsa-miR-4715-3p statt. Gemäß bestimmten Ausführungsformen findet mindestens ein Vergleich mit einer Sequenz umfassend die der miRNA hsa-miR-5011-3p statt. Gemäß bestimmten Ausführungsformen findet mindestens ein Vergleich mit einer Sequenz umfassend die miRNA hsa-miR-6778-3p statt. Gemäß bestimmten Ausführungsformen findet mindestens ein Vergleich mit einer Sequenz umfassend die miRNA hsa-miR-6833-5p statt. Gemäß bestimmten Ausführungsformen findet mindestens ein Vergleich mit einer Sequenz umfassend die miRNA hsa-miR-6873-5p statt.

Gemäß bestimmten Ausführungsformen wird RRMS in einem Patienten diagnostiziert, welche sich gewöhnlich in wiederkehrenden Schüben nach der CIS-Diagnose zeigen kann.

Gemäß bestimmten Ausführungsformen wird in mindestens 2, 3, 4, 5, 6, 7, 8, 9, 10, 11, 12 oder 13 miRNAs, die eine Sequenz umfassen, die ausgewählt ist aus der Gruppe, bestehend aus hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5, bevorzugt hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-5011-3p, hsa-miR-6778-3p, und hsa-miR-6873-5p, weiter bevorzugt hsa-miR-181d-5p, hsa-miR-194-5p, hsa-miR-627-5p, hsa-miR-148a-5p, hsa-miR-4465, hsa-miR-4699-3p, hsa-miR-5011-3p, und hsa-miR-6873-5p, und/oder die eine Sequenz umfasst, die von diesen Sequenzen um jeweils bis zu 3 Basen, bevorzugt bis zu 2 Basen, weiter bevorzugt 1 Base abweicht, eine Änderung im Expressionsniveau im Vergleich zu einem Referenzniveau beobachtet. Gemäß bestimmten Ausführungsformen wird in mindestens 2, 3, 4, 5, 6, 7, 8, 9, 10, 11, 12 oder 13 miRNAs, die eine Sequenz umfassen, die ausgewählt ist aus der Gruppe, bestehend aus hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5, bevorzugt hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-5011-3p, hsa-miR-6778-3p, und hsa-miR-6873-5p, weiter bevorzugt hsa-miR-181d-5p, hsa-miR-194-5p, hsa-miR-627-5p, hsa-miR-148a-5p, hsa-miR-4465, hsa-miR-4699-3p, hsa-miR-5011-3p, und hsa-miR-6873-5p, eine Änderung im Expressionsniveau im Vergleich zu einem Referenzniveau beobachtet. Bei einer Änderung im Expressionsniveau von mehr als einer miRNA kann hierbei das Signifikanzniveau für eine Diagnose mit einer MS-Erkrankung, insbesondere RRMS, außerordentlich gesteigert werden.

Gemäß bestimmten Ausführungsformen ist die miRNA mindestens eine, die ausgewählt ist aus der Gruppe, bestehend aus hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-4715, hsa-mir-5011, hsa-mir-6778, hsa-mir-6833, und hsa-mir-6873, bevorzugt hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-5011, hsa-mir-6778, und hsa-mir-6873, weiter bevorzugt hsa-mir-181d, hsa-mir-194-1, has-mir-194-2, hsa-mir-627, hsa-mir-148a, hsa-mir-4465, hsa-mir-4699, hsa-mir-5011, und hsa-mir-6873, und/oder mit einer Sequenz, die von dieser Sequenz um bis zu 3 Basen, bevorzugt bis zu 2 Basen, weiter bevorzugt 1 Base abweicht. Gemäß bestimmten Ausführungsformen ist die miRNA mindestens eine, die ausgewählt ist aus der Gruppe, bestehend aus hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-4715, hsa-mir-5011, hsa-mir-6778, hsa-mir-6833, und hsa-mir-6873, bevorzugt hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-5011, hsa-mir-6778, und hsa-mir-6873, weiter bevorzugt hsa-mir-181d, hsa-mir-194-1, has-mir-194-2, hsa-mir-627, hsa-mir-148a, hsa-mir-4465, hsa-mir-4699, hsa-mir-5011, und hsa-mir-6873. Diese Ausführungsform der miRNA entspricht hierbei der Stem-Loop Form bzw. Haarnadelform, welche mit der detektierten reifen Form wie in Tabelle 2 gezeigt korreliert, und welche beispielsweise in den Proben als solche enthalten sein kann. Entsprechend kann der Vergleich anstelle mit der jeweiligen reifen Form auch mit der entsprechenden Stem-Loop Form erfolgen.

**Tabelle 2: Korrelation zwischen Stem-Loop Form und reifen Form**

| **Stem-Loop Form (Haarnadelform)** | **SEQ ID NO:** | **Reife Form** | **Sequenz der reifen Form** |
|---|---|---|---|
| | | | |
| hsa-mir-194-1 | 14 | hsa-miR-194-5p | uguaacagcaacuccaugugga |
| hsa-mir-194-2 | 15 | hsa-miR-194-5p | uguaacagcaacuccaugugga |
| hsa-mir-627 | 16 | hsa-miR-627-5p | gugagucucuaagaaaagagga |
| hsa-mir-148a | 17 | hsa-miR-148a-5p | aaaguucugagacacuccgacu |
| hsa-mir-1181 | 18 | hsa-miR-1181 | ccgucgccgccacccgagccg |
| hsa-mir-1273g | 19 | hsa-miR-1273g-3p | accacugcacuccagccugag |
| hsa-mir-181d | 20 | hsa-miR-181d-5p | aacauucauuguugucggugggu |
| hsa-mir-4465 | 21 | hsa-miR-4465 | cucaaguagucugaccagggga |
| hsa-mir-4699 | 22 | hsa-miR-4699-3p | aauuuacucugcaaucuucucc |
| hsa-mir-4715 | 23 | hsa-miR-4715-3p | gugccaccuuaacugcagccaau |
| hsa-mir-5011 | 24 | hsa-miR-5011-3p | gugcauggcuguauauauaaca |
| hsa-mir-6778 | 25 | hsa-miR-6778-3p | ugccucccugacauuccacag |
| hsa-mir-6833 | 26 | hsa-miR-6833-5p | guguggaagaugggaggagaaa |
| hsa-mir-6873 | 27 | hsa-miR-6873-5p | cagagggaauacagagggcaau |

Die Nukleinsäuresequenzen der Stem-Loop Formen gemäß Tabelle 2 sind wie folgt:

14
augguguuau caaguguaac agcaacucca uguggacugu guaccaauuu
ccaguggaga ugcuguuacu uuugaugguu accaa

15
ugguucccgc ccccuguaac agcaacucca uguggaagug cccacugguu
ccaguggggc ugcuguuauc uggggcgagg gccag

16
uacuuauuac ugguagugag ucucuaagaa aagaggaggu gguuguuuuc
cuccucuuuu cuuugagacu cacuaccaau aauaagaaau acuacua

17
gaggcaaagu ucugagacac uccgacucug aguaugauag aagucagugc
acuacagaac uuugucuc

18
uccacugcug ccgccgucgc cgccacccga gccggagcgg gcuggccgc
caaggcaaga ugguggacua cagcgugugg g

19
gaggugggag gauugcuuga gucagggugg uugaggcugc aguaaguugu
gaucauacca cugcacucca gccugaguga cagagcaaga ccuugucuca

20
gucccucccc cuaggccaca gccgagguca caaucaacau ucauuguugu
cgguggguug ugaggacuga ggccagaccc accgggggau gaaugucacu
guggcugggc cagacacggc uuaaggggaa uggggac

21
cauguguccc cuggcacgcu auuugagguu uacuauggaa ccucaaguag
ucugaccagg ggacacauga

22
agcaauugga gaagauugca gaguaaguuc cugauuaaga aauggaauuu
acucugcaau cuucuccaau ugcu

23
ggggaaugaa aguuggcugc aguuaaggug gcuaaucagc ugauggugcc
accuuaacug cagccaauuc uaauucccc

24
agaugguauu gaguggaugc uguuauauau acagccaugc acucuguagu uugg-
guacac agugcauggc uguauauaua acacuaucca uucaucuuuc
agc

25
guucaagugg gaggacagga ggcaggugug guuggaggaa gcagccugaa
ccugccuccc ugacauucca cag

26
aaacggugug gaagauggga ggagaaaaau cccuguuaac uuucucucuc
cacuuccuca g

27
cccagcagag ggaauacaga gggcaaucag gacuggguca uucucucugu
cuuucucucu cag

Gemäß bestimmten Ausführungsformen wird in mindestens 2, 3, 4, 5, 6, 7, 8, 9, 10, 11, 12, 13 oder 14 miRNAs, die eine Sequenz umfassen, die ausgewählt ist aus der Gruppe, bestehend aus hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-4715, hsa-mir-5011, hsa-mir-6778, hsa-mir-6833, und hsa-mir-6873, bevorzugt hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-5011, hsa-mir-6778, und hsa-mir-6873, weiter bevorzugt hsa-mir-181d, hsa-mir-194-1, has-mir-194-2, hsa-mir-627, hsa-mir-148a, hsa-mir-4465, hsa-mir-4699, hsa-mir-5011, und hsa-mir-6873, und/oder die eine Sequenz umfassen, die von diesen Sequenzen um jeweils bis zu 3 Basen, bevorzugt bis zu 2 Basen, weiter bevorzugt 1 Base abweicht, eine Änderung im Expressionsniveau im Vergleich zu einem Referenzniveau beobachtet. Gemäß bestimmten Ausführungsformen wird in mindestens 2, 3, 4, 5, 6, 7, 8, 9, 10, 11, 12, 13 oder 14 miRNAs, die eine Sequenz umfassen, die ausgewählt ist aus der Gruppe, bestehend aus hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-4715, hsa-mir-5011, hsa-mir-6778, hsa-mir-6833, und hsa-mir-6873, bevorzugt hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-5011, hsa-mir-6778, und hsa-mir-6873, weiter bevorzugt hsa-mir-181d, hsa-mir-194-1, has-mir-194-2, hsa-mir-627, hsa-mir-148a, hsa-mir-4465, hsa-mir-4699, hsa-mir-5011, und hsa-mir-6873, eine Änderung im Expressionsniveau im Vergleich zu einem Referenzniveau beobachtet.

Gemäß einem weiteren Aspekt betrifft die Erfindung die Verwendung mindestens einer miRNA, die eine Sequenz umfasst, die ausgewählt ist aus der Gruppe, bestehend aus hsa-miR-181d-5p,hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5, bevorzugt hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-5011-3p, hsa-miR-6778-3p, und hsa-miR-6873-5p, weiter bevorzugt hsa-miR-181d-5p, hsa-miR-194-5p, hsa-miR-627-5p, hsa-miR-148a-5p, hsa-miR-4465, hsa-miR-4699-3p, hsa-miR-5011-3p, und hsa-miR-6873-5p, und/oder die eine Sequenz umfasst, die von diesen Sequenzen um jeweils bis zu 3 Basen, bevorzugt bis zu 2 Basen, weiter bevorzugt 1 Base abweicht, als diagnostischer Marker für die Diagnose von multipler Sklerose. Gemäß bestimmten Ausführungsformen wird mindestens eine miRNA, die eine Sequenz umfasst, die ausgewählt ist aus der Gruppe, bestehend aus hsa-miR-181d-5p,hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5, bevorzugt hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-5011-3p, hsa-miR-6778-3p, und hsa-miR-6873-5p, weiter bevorzugt hsa-miR-181d-5p, hsa-miR-194-5p, hsa-miR-627-5p, hsa-miR-148a-5p, hsa-miR-4465, hsa-miR-4699-3p, hsa-miR-5011-3p, und hsa-miR-6873-5p, verwendet.

Gemäß bestimmten Ausführungsformen ist hierbei die miRNA mindestens eine, die eine Sequenz aufweist, die ausgewählt ist aus der Gruppe, bestehend aus hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-4715, hsa-mir-5011, hsa-mir-6778, hsa-mir-6833, und hsa-mir-6873, bevorzugt hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-5011, hsa-mir-6778, und hsa-mir-6873, weiter bevorzugt hsa-mir-181d, hsa-mir-194-1, has-mir-194-2, hsa-mir-627, hsa-mir-148a, hsa-mir-4465, hsa-mir-4699, hsa-mir-5011, und hsa-mir-6873, und/oder die eine Sequenz aufweist, die von diesen Sequenzen um jeweils bis zu 3 Basen, bevorzugt bis zu 2 Basen, weiter bevorzugt 1 Base abweicht. Gemäß bestimmten Ausführungsformen ist hierbei die miRNA mindestens eine, die eine Sequenz aufweist, die ausgewählt ist aus der Gruppe, bestehend aus hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-4715, hsa-mir-5011, hsa-mir-6778, hsa-mir-6833, und hsa-mir-6873, bevorzugt hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-5011, hsa-mir-6778, und hsa-mir-6873, weiter bevorzugt hsa-mir-181d, hsa-mir-194-1, has-mir-194-2, hsa-mir-627, hsa-mir-148a, hsa-mir-4465, hsa-mir-4699, hsa-mir-5011, und hsa-mir-6873.

Gemäß bestimmten Ausführungsformen werden die miRNAs als Marker für RRMS verwendet.

In einem weiteren Aspekt betrifft die vorliegende Erfindung ein Kit zur Diagnose von multipler Sklerose in einer Blutprobe eines Patienten, umfassend:
Sonden und/oder Primer zur Detektion mindestens einer miRNA-Sequenz, die eine Sequenz umfasst, die ausgewählt ist aus der Gruppe, bestehend aus hsa-miR-181d-5p,hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5, bevorzugt hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-5011-3p, hsa-miR-6778-3p, und hsa-miR-6873-5p, weiter bevorzugt hsa-miR-181d-5p, hsa-miR-194-5p, hsa-miR-627-5p, hsa-miR-148a-5p, hsa-miR-4465, hsa-miR-4699-3p, hsa-miR-5011-3p, und hsa-miR-6873-5p, und/oder die eine Sequenz umfasst, die von diesen Sequenzen um jeweils bis zu 3 Basen, bevorzugt bis zu 2 Basen, weiter bevorzugt 1 Base abweicht.

Gemäß bestimmten Ausführungsformen umfasst das Kit Sonden und/oder Primer zur Detektion mindestens einer miRNA-Sequenz, die eine Sequenz umfasst, die ausgewählt ist aus der Gruppe, bestehend aus hsa-miR-181d-5p,hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5, bevorzugt hsa-miR-181d-5p, hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-5011-3p, hsa-miR-6778-3p, und hsa-miR-6873-5p, weiter bevorzugt hsa-miR-181d-5p, hsa-miR-194-5p, hsa-miR-627-5p, hsa-miR-148a-5p, hsa-miR-4465, hsa-miR-4699-3p, hsa-miR-5011-3p, und hsa-miR-6873-5p.

Gemäß bestimmten Ausführungsformen umfasst das Kit Sonden und/oder Primer zur Detektion mindestens einer miRNA-Sequenz, die eine Sequenz aufweist, die ausgewählt ist aus der Gruppe, bestehend aus hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-4715, hsa-mir-5011, hsa-mir-6778, hsa-mir-6833, und hsa-mir-6873, bevorzugt hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-5011, hsa-mir-6778, und hsa-mir-6873, weiter bevorzugt hsa-mir-181d, hsa-mir-194-1, has-mir-194-2, hsa-mir-627, hsa-mir-148a, hsa-mir-4465, hsa-mir-4699, hsa-mir-5011, und hsa-mir-6873,und/oder die eine Sequenz aufweist, die von diesen Sequenzen um jeweils bis zu 3 Basen, bevorzugt bis zu 2 Basen, weiter bevorzugt 1 Base abweicht.

Gemäß bestimmten Ausführungsformen umfasst das Kit Sonden und/oder Primer zur Detektion mindestens einer miRNA-Sequenz, die eine Sequenz aufweist, die ausgewählt ist aus der Gruppe, bestehend aus hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-4715, hsa-mir-5011, hsa-mir-6778, hsa-mir-6833, und hsa-mir-6873, bevorzugt hsa-mir-181d, hsa-mir-194-1, has-mir-194-2, hsa-mir-627, hsa-mir-148a, hsa-mir-1181, hsa-mir-1273g, hsa-mir-4465, hsa-mir-4699, hsa-mir-5011, hsa-mir-6778, und hsa-mir-6873, weiter bevorzugt hsa-mir-181d, hsa-mir-194-1, has-mir-194-2, hsa-mir-627, hsa-mir-148a, hsa-mir-4465, hsa-mir-4699, hsa-mir-5011, und hsa-mir-6873, .

Gemäß bestimmten Ausführungsformen umfasst das Kit Sonden und/oder Primer zur Detektion von 2, 3, 4, 5, 6, 7, 8, 9, 10, 11, 12, 13 oder 14 der obigen Sequenzen.

Die Sonden und/oder Primer sind hierbei nicht besonders beschränkt und können beispielsweise Oligomere umfassen, welche eine Nukleotidsequenz aufweisen, beispielsweise auch eine cDNA-Sequenz, welche komplementär zur Sequenz ist, die zu detektieren ist.

Daneben können auch weitere Sonden und/oder Primer als Kontollen vorgesehen sein, beispielsweise von weiteren miRNAs, bei denen keine Änderung des Expressionsprofils im Vergleich zu Patienten ohne MS-Erkrankung, insbesondere RRMS-Erkrankung, auftritt. Auch können Kontrollen für nichtspezifisches Binden und/oder Hybridisieren vorgesehen sein.

Die Primer und/oder Sonden sowie ggf. Kontrollen können gemäß bestimmten Ausführungsformen auf ein geeignetes Substrat aufgebracht sein.

Gemäß bestimmten Ausführungsformen umfasst das Kit weiter Enzyme und/oder Reagenzien zur Durchführung einer RT-PCR, insbesondere qRT-PCR, wobei diese nicht besonders beschränkt sind. Reagenzien können hierbei beispielsweise auch Marker, z.B. zur Fluoreszenzmarkierung und/oder Radionukleotid-Markierung, umfassen. Auch kann das Kit Reagenzien zur cDNA-Synthese aus den miRNAs vor der PCR, bevorzugt qPCR und/oder RT-PCR, insbesondere qRT-PCR, umfassen.

Gemäß bestimmten Ausführungsformen wird mit dem erfindungsgemäßen Kit RRMS diagnostiziert.

Die obigen Ausführungsformen, Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung. Insbesondere wird der Fachmann auch Einzelaspekte als Verbesserungen oder Ergänzungen zu der jeweiligen Grundform der vorliegenden Erfindung hinzufügen.

Die Erfindung wird im Anschluss anhand von beispielhaften Ausführungsformen weiter verdeutlicht, welche die Erfindung jedoch nicht einschränken.

### Beispiel 1

Es wurden in 2 Studien Blutproben von verschiedenen Patienten mit einer CIS-Diagnose bzw. RRMS-Diagnose bereitgestellt. In Studie 1 hatten 11 Patienten eine CIS- und 4 Patienten eine RRMS-Diagnose. In Studie 2 hatten 63 Patienten eine CIS- und 34 Patienten eine RRMS-Diagnose.

Aus diesen Blutproben wurden die Daten von miRNAs durch qRT-PCR, Mikroarray-Analyse und Next Generation Sequencing, wie in Keller, A., Leidinger, P., Steinmeyer, F., Stähler, C., Franke, A., Hemmrich-Stanisak, G., Ruprecht, K. (2014). Comprehensive analysis of microRNA profiles in multiple sclerosis including next-generation sequencing. Multiple Sclerosis (Houndmills, Basingstoke, England), 20(3), 295-303. doi:10.1177/1352458513496343, angegeben, bestimmt.

Aus diesen Daten wurden differentiell exprimierte miRNA zwischen CIS und RRMS Patienten berechnet. Die Daten wurden quantil normalisiert (R package "preprocessCore").

Die CIS- und RRMS-Gruppen beider Studien wurden mit den folgenden drei Kriterien verglichen:
1. p-Wert im ungepaarten t-test CIS -> RRMS < 0.05
   Logik: dys-reguliert zwischen CIS Patienten und RRMS Patienten beider Studien
2. p-Wert im ungepaarten t-test CIS1 -> CIS2 > 0.05
   Logik: ähnlich in CIS Patienten der ersten und zweiten Studie
3. p-Wert im ungepaarten t-test RRMS1 -> RRMS2 > 0.05
   Logik: ähnlich in RRMS Patienten der ersten und zweiten Studie

Mit diesen Patientengruppen wurde ein ungepaarter t-test vorgenommen. Hierbei wurden 61 miRNAs gefunden, welche entsprechend der genannten Logik unterschiedlich exprimiert sind. Die am stärksten unterschiedlich exprimierten miRNAs sind in Tabelle 3 gezeigt.

**Tabelle 3: miRNAs mit signifikant unterschiedlichem Expressionsniveau in RRMS-Patienten im Vergleich zu CIS-Patienten**

| **Reife Form** | **CIS -> RRMS** | **CIS1 -> CIS2** | **RRMS1 -> RRMS2** |
|---|---|---|---|
| hsa-miR-148a-5p | 0.00512 | 0.11160 | 0.34071 |
| hsa-miR-181d-5p | 0.00178 | 0.24502 | 0.18053 |
| hsa-miR-194-5p | 0.00033 | 0.08340 | 0.36785 |
| hsa-miR-627-5p | 0.00448 | 0.15735 | 0.51109 |
| hsa-miR-15a-5p | 0.00411 | 0.43468 | 0.06072 |
| hsa-miR-17-5p | 0.00555 | 0.14318 | 0.24316 |
| hsa-miR-126-5p | 0.00664 | 0.55909 | 0.22976 |
| hsa-miR-181a-5p | 0.00717 | 0.31625 | 0.22896 |
| hsa-miR-454-3p | 0.00725 | 0.07724 | 0.43820 |

Wie aus der Tabelle ersichtlich können die erfindungsgemäß verwendeten Marker eine Diagnose des Auftretens von RRMS in bisher als CIS-Patienten diagnostizierten Patienten ermöglichen.

### Beispiel 2

Es wurden in Studie 2 jeweils zwei zu unterschiedlichen Zeiten t1 und t2 genommene Blutproben von verschiedenen Patienten mit einer CIS-Diagnose bzw. RRMS-Diagnose, bei denen sich danach die Diagnose über die Zeit änderte oder nicht, bereitgestellt, wobei die folgenden Szenarien auftraten:
CIS --> CIS (5 Patienten)
RRMS --> RRMS (4 Patienten)
CIS --> RRMS (6 Patienten).

Aus diesen Blutproben wurden die Daten von miRNAs durch qRT-PCR, Mikroarray-Analyse und Next Generation Sequencing, wie in Keller, A., Leidinger, P., Steinmeyer, F., Stähler, C., Franke, A., Hemmrich-Stanisak, G., Ruprecht, K. (2014). Comprehensive analysis of microRNA profiles in multiple sclerosis including next-generation sequencing. Multiple Sclerosis (Houndmills, Basingstoke, England), 20(3), 295-303. doi:10.1177/1352458513496343, angegeben, bestimmt.

Aus diesen Daten wurden differentiell exprimierte miRNA zwischen CIS und RRMS Patienten berechnet. Die Daten wurden quantil normalisiert (R package "preprocessCore").

Die CIS und RRMS Gruppen sowie die CIS -> RRMS-Gruppe wurden mit den folgenden drei Kriterien verglichen:
1. p-Wert im gepaarten t-test CIS-t1 -> RRMS-t2 < 0.05
   Logik: unterschiedlich exprimiert in den beiden Blutproben von Patienten die bei t1 eine CIS- und bei t2 eine RRMS-Diagnose hatten
2. p- Wert im gepaarten t-test CIS-t1 -> CIS-t2 > 0.05
   Logik: ähnlich in den beiden Blutproben von Patienten die zu t1 und t2 eine CIS-Diagnose hatten
3. p- Wert im gepaarten t-test RRMS-t1 vs. RRMS-t2 > 0.05
   Logik: ähnlich in den beiden Blutproben von Patienten die zu t1 und t2 eine RRMS-Diagnose hatten

Mit diesen Patientengruppen wurde ein gepaarter t-test vorgenommen. Hierbei wurden 125 miRNAs gefunden, die in der Gruppe CIS-t1 --> RRMS-t2 signifikant unterschiedlich (p- Wert <0.05) exprimiert und in den anderen beiden Gruppen ähnlich exprimiert sind.

Von diesen wurden die folgenden am stärksten signifikant unterschiedlich exprimierten miRNAs in Tabelle 4 gefunden.

**Tabelle 4: miRNAs mit signifikant unterschiedlichem Expressionsniveau in RRMS-Patienten im Vergleich zu CIS-Patienten**

| **Reife Form** | **CIS-t1 -> RRMS-t2** | **CIS-t1 -> CIS-t2** | **RRMS-t1 -> RRMS-t2** |
|---|---|---|---|
| hsa-miR-1181 | 0,00139 | 0,23726 | 0,36030 |
| hsa-miR-1273g-3p | 0,00181 | 0,24681 | 0,63762 |
| hsa-miR-21d-5p | 0,00092 | 0,15330 | 0,97948 |
| hsa-miR-4465 | 0,00099 | 0,39900 | 0,60652 |
| hsa-miR-4699-3p | 0,00099 | 0,39900 | 0,60652 |
| hsa-miR-4715-3p | 0,00127 | 0,02286 | 0,73953 |
| hsa-miR-5011-3p | 0,00059 | 0,13118 | 0,68909 |
| hsa-miR-6778-3p | 0,00136 | 0,12056 | 0,78499 |
| hsa-miR-6833-5p | 0,00093 | 0,15330 | 0,97948 |
| hsa-miR-6873-5p | 0,00036 | 0,06181 | 0,93094 |

Wie aus der Tabelle 4 ersichtlich können die erfindungsgemäß verwendeten Marker eine Diagnose des Auftretens von RRMS in CIS-Patienten ermöglichen.

## Patentansprüche

1. Verfahren zur Diagnose von multipler Sklerose in einem Patienten, umfassend:
Bereitstellen einer Blutprobe eines Patienten,
Erstellen eines Expressionsprofils von miRNAs aus der Blutprobe, und
Vergleich des Expressionsniveaus mindestens einer miRNA, die eine Sequenz umfasst, die ausgewählt ist aus der Gruppe, bestehend aus hsa-miR-181d-5p,hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5p, und/oder die eine Sequenz umfasst, die von diesen Sequenzen um jeweils bis zu 3 Basen, bevorzugt bis zu 2 Basen, weiter bevorzugt 1 Base abweicht, mit einem Referenzniveau, wobei der Vergleich die Diagnose einer Erkrankung mit multipler Sklerose ermöglicht.

2. Verfahren nach Anspruch 1, wobei der Patient ein Mensch ist.

3. Verfahren nach Anspruch 1 oder 2, wobei der Patient ein Mensch ist, bei dem CIS diagnostiziert wurde.

4. Verfahren nach einem der vorigen Ansprüche, wobei die Erkrankung RRMS ist.

5. Verfahren nach einem der vorigen Ansprüche, wobei in mindestens 2, 3, 4, 5, 6, 7, 8, 9, 10, 11, 12 oder 13 miRNAs, die eine Sequenz umfasst, die ausgewählt ist aus der Gruppe, bestehend aus hsa-miR-181d-5p,hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5p, und/oder die eine Sequenz umfasst, die von diesen Sequenzen um jeweils bis zu 3 Basen, bevorzugt bis zu 2 Basen, weiter bevorzugt 1 Base abweicht, eine Änderung im Expressionsniveau im Vergleich zu einem Referenzniveau beobachtet wird.

6. Verfahren nach einem der vorigen Ansprüche, wobei die miRNA mindestens eine ist, die ausgewählt ist aus der Gruppe, bestehend aus hsa-miR-181d, hsa-miR-194-1, has-miR-194-2, hsa-miR-627, hsa-miR-148a, hsa-mir-1181, hsa-mir-1273g, hsa-mir-4465, hsa-mir-4699, hsa-mir-4715, hsa-mir-5011, hsa-mir-6778, hsa-mir-6833, und hsa-mir-6873.

7. Verfahren nach einem der vorigen Ansprüche, wobei die Erstellung des Expressionsprofils eine Nukleinsäurehybridisierung, eine Nukleinsäureamplifikation, eine Polymeraseextension, eine Sequenzierung, eine Massenspektrometrie, oder jegliche Kombination davon umfasst.

8. Verfahren nach einem der vorigen Ansprüche, wobei das Referenzniveau aus einer Vielzahl von Proben von Patienten erhalten wird, von denen eine erste Gruppe in einer ersten und zweiten Diagnose mit CIS diagnostiziert wurde, eine zweite Gruppe nach einer ersten CIS-Diagnose in einer zweiten Diagnose mit RRMS diagnostiziert wurde, und eine dritte Gruppe in einer ersten und zweiten Diagnose mit RRMS diagnostiziert wurde.

9. Verwendung einer miRNA, die eine Sequenz umfasst, die ausgewählt ist aus der Gruppe, bestehend aus hsa-miR-181d-5p,hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5p, und/oder die eine Sequenz umfasst, die von diesen Sequenzen um jeweils bis zu 3 Basen, bevorzugt bis zu 2 Basen, weiter bevorzugt 1 Base abweicht, als diagnostischer Marker für die Diagnose von multipler Sklerose.

10. Verwendung nach Anspruch 9, wobei die miRNA mindestens eine ist, die ausgewählt ist aus der Gruppe, bestehend aus hsa-miR-181d, hsa-miR-194-1, has-miR-194-2, hsa-miR-627, hsa-miR-148a, hsa-mir-1181, hsa-mir-1273g, hsa-mir-4465, hsa-mir-4699, hsa-mir-4715, hsa-mir-5011, hsa-mir-6778, hsa-mir-6833, und hsa-mir-6873.

11. Verwendung nach Anspruch 9 oder 10, wobei die miRNA als diagnostischer Marker für RRMS verwendet wird.

12. Kit zur Diagnose von multipler Sklerose in einer Blutprobe eines Patienten, umfassend:
Sonden und/oder Primer zur Detektion mindestens einer miRNA-Sequenz, die eine Sequenz umfasst, die ausgewählt ist aus der Gruppe, bestehend aus hsa-miR-181d-5p,hsa-miR-194-5p, hsa-miR-627-5p, hsa-miR-148a-5p, hsa-miR-1181, hsa-miR-1273g-3p, hsa-miR-4465, hsa-miR-4699-3p, hsa-miR-4715-3p, hsa-miR-5011-3p, hsa-miR-6778-3p, hsa-miR-6833-5p, und hsa-miR-6873-5p, und/oder die eine Sequenz umfasst, die von diesen Sequenzen um jeweils bis zu 3 Basen, bevorzugt bis zu 2 Basen, weiter bevorzugt 1 Base abweicht.

13. Kit nach Anspruch 12, weiter umfassend Enzyme und/oder Reagenzien zur Durchführung einer RT-PCR.

14. Kit nach Anspruch 12 oder 13, wobei RRMS diagnostiziert wird.


SEQUENCE LISTING

<110>  Siemens Healthcare GmbH

<120>  Diagnostische miRNA Signaturen in MS und CIS Patienten

<130>  201517211

<160>  27

<170>  PatentIn version 3.5

<210>  1
<211>  22
<212>  RNA
<213>  Homo sapiens

<400>  1
uguaacagca acuccaugug ga                                                22


<210>  2
<211>  22
<212>  RNA
<213>  Homo sapiens

<400>  2
gugagucucu aagaaaagag ga                                                22


<210>  3
<211>  22
<212>  RNA
<213>  Homo sapiens

<400>  3
aaaguucuga gacacuccga cu                                                22


<210>  4
<211>  21
<212>  RNA
<213>  Homo sapiens

<400>  4
ccgucgccgc cacccgagcc g                                                 21


<210>  5
<211>  21
<212>  RNA
<213>  Homo sapiens

<400>  5
accacugcac uccagccuga g                                                 21


<210>  6
<211>  23
<212>  RNA
<213>  Homo sapiens

<400>  6
aacauucauu guugucggug ggu                                               23


<210>  7
<211>  22

<212>  RNA
<213>  Homo sapiens

<400>  7
cucaaguagu cugaccaggg ga                                                22


<210>  8
<211>  22
<212>  RNA
<213>  Homo sapiens

<400>  8
aauuuacucu gcaaucuucu cc                                                22


<210>  9
<211>  23
<212>  RNA
<213>  Homo sapiens

<400>  9
gugccaccuu aacugcagcc aau                                               23


<210>  10
<211>  22
<212>  RNA
<213>  Homo sapiens

<400>  10
gugcauggcu guauauauaa ca                                                22


<210>  11
<211>  21
<212>  RNA
<213>  Homo sapiens

<400>  11
ugccucccug acauuccaca g                                                 21


<210>  12
<211>  22
<212>  RNA
<213>  Homo sapiens

<400>  12
guguggaaga ugggaggaga aa                                                22


<210>  13
<211>  22
<212>  RNA
<213>  Homo sapiens

<400>  13
cagagggaau acagagggca au                                                22


<210>  14
<211>  85
<212>  RNA
<213>  Homo sapiens

<400>  14
augguguuau caaguguaac agcaacucca uguggacugu guaccaauuu ccaguggaga      60
Seite 2

ugcuguuacu uuugaugguu accaa                                          85


<210>  15
<211>  85
<212>  RNA
<213>  Homo sapiens

<400>  15
ugguucccgc ccccuguaac agcaacucca uguggaagug cccacugguu ccaguggggc      60

ugcuguuauc uggggcgagg gccag                                          85


<210>  16
<211>  97
<212>  RNA
<213>  Homo sapiens

<400>  16
uacuuauuac ugguagugag ucucuaagaa aagaggaggu gguuguuuuc cuccucuuuu      60

cuuugagacu cacuaccaau aauaagaaau acuacua                             97


<210>  17
<211>  68
<212>  RNA
<213>  Homo sapiens

<400>  17
gaggcaaagu ucugagacac uccgacucug aguaugauag aagucagugc acuacagaac      60

uuugucuc                                                             68


<210>  18
<211>  81
<212>  RNA
<213>  Homo sapiens

<400>  18
uccacugcug ccgccgucgc cgccacccga gccggagcgg gcugggccgc caaggcaaga      60

ugguggacua cagcgugugg g                                              81


<210>  19
<211>  100
<212>  RNA
<213>  Homo sapiens

<400>  19
gaggugggag gauugcuuga gucagggugg uugaggcugc aguaaguugu gaucauacca      60

cugcacucca gccugaguga cagagcaaga ccuugucuca                         100


<210>  20
<211>  137
<212>  RNA
<213>  Homo sapiens

<400>  20
guccccuccc cuaggccaca gccgagguca caaucaacau ucauuguugu cgguggguug      60

ugaggacuga ggccagaccc accgggggau gaaugucacu guggcugggc cagacacggc     120

uuaaggggaa uggggac                                                    137

<210>  21
<211>  70
<212>  RNA
<213>  Homo sapiens

<400>  21
cauguguccc cuggcacgcu auuugagguu uacuauggaa ccucaaguag ucugaccagg      60

ggacacauga                                                             70

<210>  22
<211>  74
<212>  RNA
<213>  Homo sapiens

<400>  22
agcaauugga gaagauugca gaguaaguuc cugauuaaga aauggaauuu acucugcaau      60

cuucuccaau ugcu                                                        74

<210>  23
<211>  79
<212>  RNA
<213>  Homo sapiens

<400>  23
ggggaaugaa aguuggcugc aguuaaggug gcuaaucagc ugauggugcc accuuaacug      60

cagccaauuc uaauucccc                                                   79

<210>  24
<211>  103
<212>  RNA
<213>  Homo sapiens

<400>  24
agaugguauu gaguggaugc uguuauauau acagccaugc acucuguagu uuggguacac      60

agugcauggc uguauauaua acacuaucca uucaucuuuc agc                       103

<210>  25
<211>  73
<212>  RNA
<213>  Homo sapiens

<400>  25
guucaagugg gaggacagga ggcaggugug guuggaggaa gcagccugaa ccugccuccc      60

ugacauucca cag                                                         73

<210>  26
<211>  61
<212>  RNA
<213>  Homo sapiens

<400>  26
aaacggugug gaagauggga ggagaaaaau cccuguuaac uuucucucuc cacuuccuca      60

g                                                                      61

<210>  27
<211>  63
<212>  RNA
<213>  Homo sapiens

<400>  27
cccagcagag ggaauacaga gggcaaucag gacuggguca uucucucugu cuuucucucu      60

cag                                                                    63